# EUROPEAN PATENT APPLICATION

(11) **EP 1 241 253 A1**
(43) Date of publication of application: **18.09.2002**
(21) Application number: 00970095.6
(22) Date of filing: 26.10.2000
(51) Int. Cl.: C12N 15/12, C12N 15/54, C12N 9/12, C12N 1/15, C12N 1/19, C12N 1/21, C12N 5/10

(54) **HUMAN PROTEINS BINDING TO HUMAN TYROSINE KINASE Hck AND GENES ENCODING THE SAME**

(30) Priority: 29.10.1999 JP 30995799
(71) Applicant: SSP Co., Ltd., Chuo-ku, Tokyo 103-8481 (JP)
(72) Inventor: TANIYAMA, Tadayoshi, Yokohama-shi, Kanagawa 231-0864 (JP); NARITA, Tadashi, Inba-gun, Chiba 285-0905 (JP)
(74) Representative: Walton, Seán Malcolm
(86) International application number: JP0007500
(87) International publication number: WO01032869

(57) **Abstract**

A novel protein having an activity to bind to human tyrosine kinase Hck is disclosed. A representative example of the protein according to the present invention has an amino acid sequence shown in SEQ ID NO: 1 or SEQ ID NO: 12. Nucleic acids encoding these proteins are also disclosed. The protein according to the present invention has a property to bind to human tyrosine kinase Hck and has a function of promoting apoptosis by stimulation with tumor necrosis factor.

## Description

### Technical Field

The present invention relates to a human protein which binds to human tyrosine kinase Hck, and a gene encoding the same.

### Background Art

Various signals from outside of the cells are transmitted into the cells mediated receptors on the cell surfaces. The information is transmitted into the nuclei by signal transduction system including various signal-transducing molecules to activate transcription factors. As a result, expression of a group of genes is induced or suppressed. A number of tyrosine kinases existing in the cells have been discovered, and it has been revealed that most of them function as intracellular signal-transducing molecules. Tyrosine kinases are grouped into receptor type and non-receptor type, and the non-receptor type tyrosine kinases are grouped into membrane-bound type and cytoplasm type. Tyrosine kinases belonging to Src family are known 9 factors in all (i.e., Src, Yes, Yrk, Fgr, Fyn, Lyn, Lck, Blk and Hck). These are tyrosine kinases consisting of about 500 amino acids, having molecular weights of about 60,000 and are membrane-bound type tyrosine kinases which do not penetrate the cell membranes but bound to intracytoplasmic membranes. The basic structures of these tyrosine kinases belonging to Src family are similar, and any of them has an SH (Src homology) 2 domain and a SH3 domain, as well as a protein kinase domain. SH2 domain and SH3 domain participate in binding between proteins. SH2 domain recognizes a sequence containing a phosphorylated tyrosine residue and specifically binds thereto. On the other hand, SH3 domain specifically binds to a sequence which is rich in proline, and is thought to phosphorylate the target protein by the protein kinase domain. It is thought that the SH2 domain and SH3 domain play important roles in mediating protein-protein interactions in the diverse signal transduction routes.

Hck is one of the tyrosine kinases belonging to Src family, and is assumed to be a molecule participating in signal transduction (ZIEGLER, S.F. et al., Molecular and Cellular Biology, 7, 2276-2285 (1987)). It has been reported that Hck associates with the subunit (gp130) of the LIF (leukemia inhibitory factor)/IL-6 (interleukin 6) receptor and participates in the signal transduction route (Emist M.,ra et al., The EMBO Journal, 13, 1574-1584(1994)); and that Hck associates with FcγRII of monocytic THP-1 cell (Ghazizadeh, S., et al., Journal of Biological Chemistry, 269, 8878-8884 (1994)). However, including these, detailed functions of Hck in the signal transduction have not yet been clarified. Recently, the role of Hck in infection by HIV has been drawing attention because the binding affinity of nef protein of AIDS virus (HIV) to the SH3 domain of human Hck is about 10 times higher than those to SH3 domains of other Src type tyrosine kinases (Lee C-H et al., EMBO Journal 14, 5006-5015 (1995)). Moreover, Hck existing in the secretory granules in neutrophiles may participate in the signal transduction after phagocytosis of infectious bacterial cells by the granulocytes (Welch, H., and Maridonneau-Parini, I., Journal of Biological Chemistry, 272, 102-109 (1997)).

A protein having an activity to bind to human intracellular tyrosine-phosphorylating enzyme Hck and a gene encoding the same are thought to be useful for the study of signal transduction in which Hck participates, for the study of behavior of nef protein of HIV and the like.

### Disclosure of the Invention

An object of the present invention is to provide a protein having an activity to bind to human intracellular tyrosine-phosphorylating enzyme Hck and a gene encoding the same.

The present inventors intensively studied to succeed in isolating a DNA encoding a protein having an activity to bind to human intracellular tyrosine-phosphorylating enzyme Hck, thereby completing the present invention.

That is, the present invention provides a protein which is the following (a) or (b):
(a) a protein having the amino acid sequence shown in SEQ ID NO:1;
(b) a protein having the same amino acid sequence as shown in SEQ ID NO: I, except that one or several amino acids are deleted, substituted, inserted or added, which protein has an activity to bind to human tyrosine kinase Hck.

The present invention also provides a protein having the amino acid sequence which has a homology of not less than 80% to the amino acid sequence shown in SEQ ID NO: 1 or a region therein, which has an activity to bind to human tyrosine kinase Hck. The present invention also provides a nucleic acid encoding the protein according to the present invention. The present invention further provides a recombinant vector containing the nucleic acid according to the present invention, which can express the protein according to the present invention in a host cell. The present invention still further provides a transformant transformed with the recombinant vector according to the present invention, which produces the protein according to the present invention.

Since the protein according to the present invention has a function to bind to human tyrosine kinase Hck, it is useful for clarification of the mechanism of the signal transduction by human Hck, and clarification of the role of human Hck in HIV infection. Moreover, it is useful for the development of therapeutic agents against the diseases and infectious diseases in which human Hck participates. Moreover, since the protein according to the present invention has a function to accelerate the apoptosis by tumor necrosis factor (TNF-α)-stimulated, it is useful as an anti-tumor activity-enhancing agent for tumor necrosis factor.

### Brief Description of the Drawings

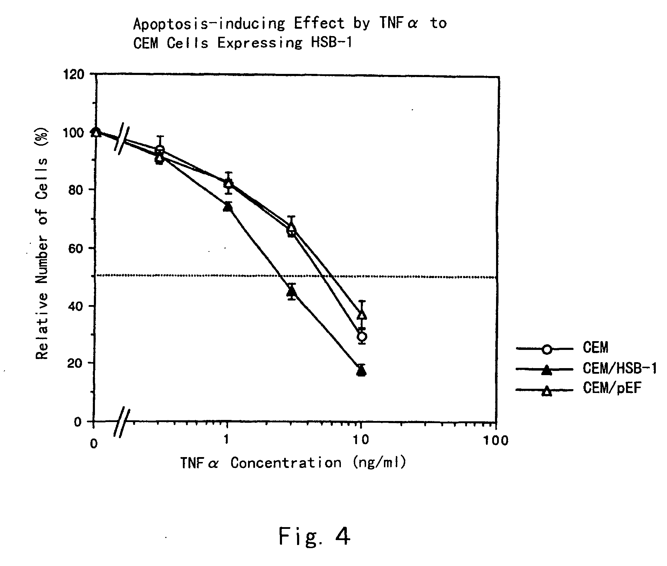

Fig. 1 is a schematic view showing the electrophoretic pattern that shows the results of expression of HSB-1 in *E. coli*, which is a protein according to one example of the present invention.
   - Lane 1:: Molecular Weight Markers
   - Lane 2:: Cell Extract before Addition of IPTG
   - Lane 3: Cell Extract after 4 Hours from Addition of IPTG
   - Lane 4:: Purified HSB-1
Fig. 2 is a schematic view showing the electrophoretic pattern which shows that the HSB-1 bound to human Hck is immunoprecipitated with human Hck, the human Hck and HSB-1 being co-expressed in the cells.
   - Lane 1:: Molecular Weight Markers
   - Lane 2:: HSB-1 was detected after immunoprecipitation of Human Hck
   - Lane 3:: Control
Fig. 3 is a schematic view showing the electrophoretic patterns which show that HSB-1 and HSB -1(70-404) each of which was bound to human Hck were immunoprecipitated, and that HSB-1(1-131) was not immunoprecipitated with human Hck, the human Hck and HSB-1 or its partial sequence HSB-1(1-131) or HSB-1(70-404) being co-expressed in the cells.
   - Lane 1:: Immunoprecipitate obtained by immunoprecipitating cell extract of the cells transformed with a control vector pcDNA4/HisMaxC and Hck/pFLAG-CMV2, with anti-human Hck antibody
   - Lane 2:: Immunoprecipitate obtained by immunoprecipitating cell extract of the cells co-expressing HSB-1 and human Hck, with anti-human Hck antibody
   - Lane 3:: Immunoprecipitate obtained by immunoprecipitating cell extract of the cells co-expressing HSB-1(1-131) and human Hck, with anti-human Hck antibody
   - Lane 4:: Immunoprecipitate obtained by immunoprecipitating cell extract of the cells co-expressing HSB-1(70-404) and human Hck, with anti-human Hck antibody
   - Lane 5:: Cell extract of the cells transformed with a control vector pcDNA4/HisMaxC and Hck/pFLAG-CMV2
   - Lane 6:: Cell extract of the cells co-expressing HSB-1 and human Hck
   - Lane 7:: Cell extract of the cells co-expressing HSB-1(1-131) and human Hck
   - Lane 8:: Cell extract of the cells co-expressing HSB-1(70-404) and human Hck
Fig. 4 shows apoptosis-inducing effect by TNF-α to the CEM cells expressing HSB-1.

### Best Mode for Carrying out the Invention

The proteins according to the present invention are protein molecules which bind to human tyrosine kinase Hck (this may be hereinafter referred to as "human Hck" for short) among the human Src family tyrosine kinases. A representative example thereof is HSB-1 obtained in Examples below. The nucleic acids according to the present invention are the nucleic acids encoding the above-mentioned proteins according to the present invention, which were created by the screening or the like using the so called yeast two-hybrid system from the cDNA library prepared from a tissue such as human placenta. A representative example thereof is the nucleic acid (hereinafter also referred to as "HSB-1DNA') encoding HSB-1, which was obtained in Examples below. The nucleic acids according to the present invention were cloned as described below.

Although the nucleic acids according to the present invention were created by the method described below, since the nucleotide sequences were determined by the present invention, the nucleic acids may easily be prepared, based on the finding by the present invention, by RT-PCR or the like using the human placenta cDNA library as templates.

### 1. Cloning of HSB-1 DNA

### (1) Obtainment of cDNA Library

Examples of the source of mRNAs include tissues such as human placenta. The source may also be a cell line established from such a tissue. Preparation of mRNAs may be carried out by a conventional method. For example, the above-mentioned tissue or cells are treated with guanidine reagent to obtain total RNA, and the obtained total RNA is subjected to affinity column method or batch method to obtain poly(A+)RNAs (mRNAs). The poly(A+)RNAs may be further fractionated by sucrose density gradient centrifugation or the like.

Using the thus obtained mRNAs as templates, single-stranded cDNAs are synthesized, and then double-stranded cDNAs are prepared therefrom, followed by inserting the resultant in an appropriate vector to prepare recombinant plasmids. *E. coli* or the like is transformed with the recombinant plasmids to obtain cDNA library. Alternatively, commercially available cDNA libraries (such as those from CLONTECH or the like) may also be used.

### (2) Construction of Bait Plasmid pAS2-1

From the cDNA library obtained as described in 1 above, plasmids for screening the desired clone are prepared as follows:

The plasmid may be constructed by, for example, ligating the DNA encoding the entire human Hck with the DNA encoding the DNA-binding domain of GAL4 to prepare a chimeric DNA, and by ligating the chimeric DNA with a bait plasmid pAS2-1. Alternatively, the plasmid may also be constructed by ligating to the DNA encoding the DNA-binding domain of GAL4 the DNAs encoding SH2 domain and SH3 domain respectively, which domains are thought to play important roles in the protein-protein interactions in signal transduction routes, together or separately to prepare a chimeric DNA, and by ligating the chimeric DNA with a bait plasmid pAS2-1.

### (3) Screening

Then the cDNA library is subjected to screening using the above-described plasmid. For the screening, yeast two-hybrid system may be used. Yeast two-hybrid system is an experiment system which enables detection of interactions between proteins in yeast cells. By this system, the cDNA encoding a protein which interacts with the target protein (bait) can be screened. The transformants transformed with the bait plasmid may be selected in a tryptophan(-) medium. By transforming a cell with the cDNA library which expresses a fusion protein between the above-mentioned protein and the transcription-activating domain of GAL4, the transformants can grow in a tryptophan(-) and leucine(-) medium. If the fusion protein originated from the bait plasmid and a fusion protein originated from the library are bound, HIS3 gene and LacZ gene, which are reporter genes, are transcribed, so that the positive clones can grow in a tryptophan(-), leucine(-) and histidine(-) medium, and have β-galactosidase activity. Thus, the positive clones may be selected based on the growth in a selection medium which does not contain histidine, tryptophan and leucine, and on the β-galactosidase activity.

### (4) Determination of Nucleotide Sequence

The obtained clones are then sequenced. Sequencing may be carried out by a known method such as Maxam Gilbert method or dideoxy method, and is usually carried out by using an automatic sequencer. Full length cDNA sequence may be determined from the thus obtained nucleotide sequence by 5'-RACE (Rapid Amplification of cDNA Ends) method, 3'-RACE method, oligocap method or the like. The nucleotide sequence of HSB-1DNA which is an example of the present invention is shown in SEQ ID NO:2, and the amino acid sequence of HSB-1 is shown in SEQ ID NO:1. As long as the protein having the amino acid sequence has the activity to bind to human Hck, the protein may be modified such, for example, that one or several amino acids may be deleted, substituted or added. For example, a mutant shown in SEQ ID NO: 12 wherein one amino acid is substituted (the 121st proline is substituted with leucine) is exemplified.

### 2. Construction of Recombinant Vector and Transformant

### (1) Construction of Recombinant Vector

The recombinant vector according to the present invention may be obtained by ligating and inserting the HSB-1DNA according to the present invention in an appropriate vector. The vector which the HSB-1DNA according to the present invention is ligated to and inserted in is not restricted as long as it can replicate in a host, and may be, for example, a plasmid DNA, phage DNA or the like. The plasmid DNA may be prepared by alkaline extraction method from *E. coli, Agrobacterium* or the like. Commercially available plasmids such as those available from Clontech, Takara Shuzo, Amersham-Pharmacia Biotech or the like may be employed.

Examples of the phage DNA include M13mp8, λgt11 and the like. Insertion of the DNA according to the present invention may be attained by, for example, first inserting the purified DNA into a restriction site or a multicloning site of an appropriate vector DNA and then ligating the DNA with the vector DNA.

It is necessary that the DNA according to the present invention be inserted into the vector such that the function of the DNA is exerted. Therefore, the vector may contain, in addition to a promoter and the DNA according to the present invention, a terminator, ribosome-binding sequence and the like. Since a number of expression vectors containing these are commercially available, the recombinant vector according to the present invention, which express the protein of the present invention, may easily be prepared by inserting the DNA fragment of the present invention into the multicloning site of such a vector.

### (2) Preparation of Transformants (or Transformed Cells)

The transformants according to the present invention may be obtained by introducing the recombinant vector for expressing the gene of the present invention into a host such that the desired gene is expressed. As the host, any host may be employed as long as the DNA of the present invention can be expressed therein. Examples of the host include bacteria such as *Escherichia coli* and *Bacillus subtilis*; yeasts such as *Saccharomyces cerevisiae*; animal cells such as COS cell and CHO cell; and insect cells (Sf9).

The method for introducing the recombinant vector into a bacterium is not restricted and any method may be employed as long as it can transfer a DNA to the bacterium. For example, a method utilizing calcium ion or electroporation method may be employed. Examples of the method for introducing the recombinant vector into a yeast include electroporation method, lithium acetate method and the like. Examples of the method for introducing the recombinant vector into an animal cell include electroporation method, calcium phosphate method and the like.

### 3. Production of HSB-1

The recombinant protein HSB-1 according to the present invention may be obtained by culturing the above-described transformants in a medium and by recovering the protein from the culture. Culturing the transformants in a medium may be carried out by an ordinary method employed for culturing the respective host. As the medium for culturing a microorganism such as *E. coli* or yeast may be a natural medium or a synthetic medium as long as it contains metabolizable carbon source, nitrogen source, inorganic salts and the like, and as long as the culturing of the transformants may be effectively carried out. Culturing is usually carried out under aerobic condition such as culturing under shaking or culturing with bubbling under stirring. In cases where a microorganism transformed with an expression vector utilizing an inducible promoter is cultured, an inducer may be added to the medium as required. For example, in cases where a microorganism transformed with an expression vector utilizing Lac promoter, isopropyl-β-D-thiogalactopyranoside (IPTG) or the like may be added to the medium.

As the medium for culturing transformants obtained using animal cells as hosts, RPMI1640 medium, DMEM medium or these medium to which fatal calf serum or the like is added may be employed. The culturing is usually carried out in an atmosphere containing 5% carbon dioxide gas.

After the culture, HSB-1 is extracted after disruption of the cells when the HSB-1 according to the present invention is produced in the cells. In cases where HSB-1 according to the present invention is secreted, HSB-1 of the present invention may be isolated and purified by one or more of general methods used for isolating and purifying proteins, after removing the cells from the culture.

By the method outlined above and which will be described concretely in Examples below, a protein having the amino acid sequence shown in SEQ ID NO: 1 was isolated. In general, it is well-known in the art that there are cases wherein the physiological activity of a physiologically active protein is retained even if the amino acid sequence of the protein is modified such that a small number of amino acids are deleted, substituted, inserted or added. Therefore, a protein having the same amino acid sequence as shown in SEQ ID NO: 1 except that one or more amino acids are deleted, substituted, inserted or added, which protein has an activity to bind to human tyrosine kinase Hck, and which protein has an amino acid sequence having a homology of not less than 80% (the protein may be hereinafter referred to as "modified protein" for convenience) as well as a nucleic acid encoding the modified protein (the nucleic acid may be hereinafter referred to as "modified nucleic acid" for convenience) is also within the scope of the present invention. As will be proved in Examples below, the region in HSB-1, which binds to human tyrosine kinase Hck is the region consisting of the 130th to 404th amino acids from the N-terminal. Therefore, a protein having the same amino acid sequence as that of a region required for the binding to Hck, and a protein having the same amino acid sequence as that of a region required for the binding to Hck except that one or more amino acids are deleted, substituted, inserted or added, which has an activity to bind to human tyrosine kinase Hck, and which protein has an amino acid sequence having a homology of not less than 80% are included in the above-mentioned modified protein, and are within the scope of the present invention. The amino acid sequence of such a modified protein has a homology of not less than 80%, preferably not less than 90%, still more preferably not less than 95%, and still more preferably not less than 98% to the amino acid sequence shown in SEQ ID NO: 1 or a region therein. Any of the nucleic acids encoding the proteins (including the above-mentioned modified proteins) according to the present invention are also within the scope of the present invention. The modified nucleic acid has a nucleotide sequence having a homology of preferably not less than 70%, more preferably not less than 85%, still more preferably not less than 90%, still more preferably not less than 95% to the nucleotide sequence shown in SEQ ID NO:2 or a region therein. The modified nucleic acid may preferably be one which hybridizes with the nucleic acid having the nucleotide sequence shown in SEQ ID NO:2 under stringent conditions (i.e., hybridization is performed at 60 to 65°C using a common hybridization solution such as 5 x Denhardt's reagent, 6 x SSC, 0.5% SDS). The homology of amino acid sequences and nucleotide sequences may easily be calculated by a well-known computer software such as FASTA.

### 4. Function of HSB-1

### (1) Structure of HSB-1 and Binding to Hck

HSB-1 has a function to bind to human tyrosine kinase Hck, and is thought to play a role in transmitting signals from Hck to the next protein. Structural analysis of the amino acid sequence revealed that the protein has an SH3 domain in the N-terminal region. As described in Examples below, HSB-1 is thought to bind to the SH3 domain of Hck at the region of 130th to 404th amino acids in the amino acid sequence shown in SEQ ID NO:12.

### (2) Function to Promote Apoptosis by Stimulation by Tumor Necrosis Factor (TNF-α)

As will be concretely described in Examples below, CEM A301 cells (hereinafter referred to as "CEM cells" for short) transformed with HSB-1/pEF/V5-HisC constantly produces HSB-1. On the other hand, tumor necrosis factor (TNF-α) is known to induce apoptosis of cells by the stimulation thereof. Apoptosis of the transformed CEM cell which overproduces HSB-1 is induced by TNF-stimulated with 1/5 to 1/10-fold concentration of TNF of that required for inducing apoptosis for the wild type CEM cell or CEM cell transformed with pEF/V5-HisC. That is, HSB-1 participates in the signal transduction to reach apoptosis induced by TNF-α, and promotes it.

### Examples

The present invention will now be described more concretely by way of examples thereof. It should be noted that the present invention is not restricted to the Examples below.

### Example 1 Cloning of HSB-1DNA

### (1) cDNA Library

A commercially available cDNA library (CLONTECH) was used.

### (2) Preparation of Plasmid

A DNA encoding SH2 domain and SH3 domain of human Hck [62nd to 217th amino acids in the amino acid sequence of human Hck (SEQ ID NO: 11)] was amplified by PCR using a PCR kit (Advantage PCR Kits) commercially available from CLONTECH. As the primers, the following was used:
- Sense Primer:: SEQ ID NO: 3
- Antisense Primer: SEQ ID NO:4

PCR was carried out by firstly conducting the reaction at 94°C for 1 minute, then repeating a cycle of 94°C for 1 minute, 58°C for 1 minute and 72°C for 1 minute 30 times, and finally conducting the reaction at 72°C for 10 minutes.

The PCR product was cloned into a plasmid pCR2.1 using TA Cloning Kits (Invitrogen) and the resulting plasmid was digested with *Eco* RI/*Sal* I to obtain a fragment with a size of about 500 bp containing the SH2 domain and SH3 domain of human Hck. The fragment was ligated to pAS-2 vector digested with *Eco* RI/*Sal* I (the obtained vector is hereinafter referred to as "Hck(SH2+SH3)/pAS-2"). *E. coli* MC1061rec⁻ was transformed with Hck(SH2+SH3)/pAS-2 by a conventional method, and the plasmid was purified by the alkali method and then by the cesium chloride/ethidium bromide buoyant density centrifugation method.

### (3) Screening

Yeast Y190 strain was transformed with the plasmid Hck(SH2+SH3)/pAS-2 which is used as the bait by the lithium acetate method. The transformation was carried out by using MATCHMAKER Two-Hybrid System kit commercially available from CLONTECH. Transformants were selected on a tryptophan(-) medium. The transformants were further transformed with a cDNA library (human placenta MATCHMAKER cDNA library commercially available from CLONTECH) which can express the protein in the form of a fusion protein with the transcription-activating domain of GAL4. The positive clones of the transformants were selected by selecting the clones (150 clones) which grew on a medium of tryptophan(-), leucine(-) and histidine(-), supplemented with 3-aminotriazole (25 mM), and by selecting the clones (51 clones) which showed β-galactosidase activity. The positive clones were cultured in a medium supplemented with cycloheximide (1 µg/ml), and the plasmids were purified from the grown clones (this was carried out by using MATCHMAKER Two-Hybrid System kit commercially available from CLONTECH). *E. coli* was transformed with the obtained plasmids and the plasmids were purified therefrom. The regions originated from the cDNA library in the plasmids were sequenced by the dye terminator method using a DNA sequencer (373A type) commercially available from Perkin-Elmer. As the primers for sequencing, those included in the kit were used. The obtained nucleotide sequences were subjected to homology search using a database GENETYX. As a result, a clone (Hck-c-58/pACT2) containing a novel sequence which is not identical to any reported genes was discovered.

Since the novel sequence contained in Hck-c-58/pACT2 lacks a part of the N-terminal region of HSB-1 gene, full length HSB-1 gene was obtained from human placenta cDNA λgt11 library (CLONTECH) as follows. The novel sequence contained in Hck-c-58/pACT2 was recovered by cleaving the plasmid with *Eco* RI/*Xho* I and purified. A radio-labelled probe labelled with ³²P-CTP was prepared by the random primer method based on the purified fragment. Human placenta cDNA λgt11 library was sown on LB agar medium together with *E. coli* (Y1090r⁻) to allow the *E. coli* to form plaques. The plaques were transferred to a nitrocellulose membrane, and autoradiography was carried out using the above-described radio-labelled probe after UV-crosslinking. The positive plaques were collected by scraping and diluted, followed by repeating the same screening to obtain a number of positive clones. The cDNA library inserts in the obtained λgt11 phages were amplified by PCR (sense primer: SEQ ID NO: 5, antisense primer: SEQ ID NO: 6; the reaction condition was 94°C for 1 minute, then repeating 30 times a cycle of 94°C for 30 seconds and 68°C for 4 minutes, and finally 68°C for 4 minutes), and the PCR products containing the N-terminal sequence of HSB-1 were cloned to pCR2.1. The insert regions were sequenced by the dye terminator method, and the nucleotide sequence of the full length of HSB-1 was determined based on the combination of the determined sequences and the sequence of the Hck-c-58/pACT2. The nucleotide sequence of HSB-1 is shown in SEQ ID NO: 2. The amino acid sequence encoded by the nucleotide sequence shown in SEQ ID NO: 2 is shown in SEQ ID NO: 1.

The longest PCR product in the positive λgt11 phage clones was cloned to the plasmid pCR2.1 using TA Cloning Kits (Invitrogen) and the insert region was sequenced by the dye terminator method to obtain a nucleotide sequence which is the same as the nucleotide sequence of HSB-1 shown in SEQ ID NO: 2 except for only one base (the 362nd cytosine(C) was substituted with thymine(T)). This nucleotide sequence is shown in SEQ ID NO: 13. The amino acid sequence encoded by the nucleotide sequence shown in SEQ ID NO: 13 is shown in SEQ ID NO: 12 (the 121st proline is substituted with leucine).

### Example 2 Construction of Recombinant Vectors, Preparation of Transformants and Expression of HSB-1

To prepare a recombinant vector containing HSB-1DNA, the cDNA (SEQ ID NO: 13) encoding HSB-1 was synthesized by PCR method. The following primers were used, and the composition of the PCR mixture was the same as described above.
- Sense Primer:: SEQ ID NO: 7
- Antisense Primer: SEQ ID NO: 8

The PCR was carried out by conducting the reaction at 94°C for 30 seconds, then repeating 30 times a cycle of 94°C for 30 seconds, 65°C for 30 seconds and 72°C for 2 minutes, and then finally conducting the reaction at 72°C for 3 minutes. The PCR product was cloned to the plasmid pCRII using TA Cloning Kits (Invitrogen) and the resulting plasmid was cleaved with *Eco* RI/*Bam* HI, followed by insertion into an expression vector pGEX-6P-1 (Amersham Pharmacia Biotech). With the obtained expression vector, *E. coli* BL21-CodmPlus-R1L (Stratagene) was transformed.

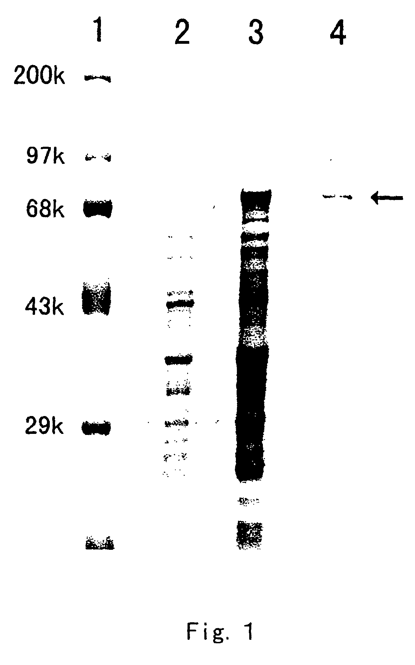

Expression of HSB-1 was carried out by culturing the thus transformed *E. coli* in LB medium (supplemented with ampicillin). To 10 ml of the medium, 0.4 ml of preculture was inoculated and the *E. coli* was cultured at 32°C for 4 hours under shaking. To this culture, 0.1 ml of 100 mM isopropyl-thio-β-D-galactoside (IPTG) was added, and the culture was continued for another 4 hours. Cells were collected from 1 ml of the culture immediately before adding IPTG and from 1 ml of the culture 4 hours after the addition of IPTG, respectively, and the proteins in the cells were analyzed by SDS-polyacrylamide gel electrophoresis. The patterns of the stained proteins after the electrophoresis are shown in Fig. 1. A fusion protein between HSB-1 and GST is expressed at a molecular weight of about 70,000.

### Example 3 Binding of HSB-1 to Human Hck

Binding between HSB-1 and human Hck was confirmed by the following method.

By the similar method to the method for preparation of the expression vector in Example 2, cDNA (SEQ ID NO: 13) encoding HSB-1 was inserted into the expression vector pEF/V5-HisC (Invitrogen) (HSB-1/pEF/V5-HisC).

On the other hand, cDNA encoding human Hck was synthesized by the PCR method. The following primers were used, and the composition of the PCR mixture was the same as described above.
- Sense Primer:: SEQ ID NO: 9
- Antisense Primer: SEQ ID NO: 10

The PCR was carried out by incubating the mixture at 94°C for 30 seconds, then repeating 30 times a cycle of 94°C for 30 seconds, 65°C for 30 seconds and 72°C for 3 minutes, and then finally at 72°C for 3 minutes. The PCR product was cloned to the plasmid pCRII using TA Cloning Kits (Invitrogen) and the resulting plasmid was cleaved with *Eco* RI/*Xho* I, followed by insertion into an expression vector pFLAG-CMV2 (Eastman Kodak) cleaved with *Eco* RI/*Sal* I (Hck/pFLAG-CMV2).

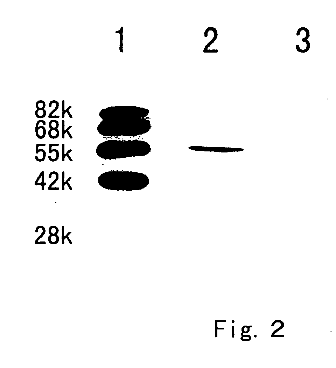

Then 293T cells were co-transformed with the two plasmids, HSB-1/pEF/V5-HisC and Hck/pFLAG-CMV2 by the calcium phosphate method (using a kit commercially available from Prime). As a control, the cells were co-transformed with HSB-1/pEF/V5-HisC and pFLAG-CMV2. After culturing the cells for 2 days, cells were collected and cell lysate solutions were prepared. The solutions were subjected to immunoprecipitation with anti-human Hck antibody, and the precipitated product was separated by SDS-polyacrylamide gel electrophoresis, followed by Western blotting and detection with anti-V5 antibody. The results of the detection are shown in Fig. 2. Since HSB-1 was detected with anti-V5 antibody, it was confirmed that HSB-1 (labelled with V5) co-expressed with human Hck bound to human Hck and immunoprecipitated with human Hck.

### Example 4 Deduction of Site in Hck, Which Binds to HSB-1

By a method similar to the method described in Example 1(2), DNAs encoding the SH2 domain (121st to 217th amino acids of the amino acid sequence (SEQ ID NO: 11) of human Hck), and the SH3 domain (62nd to 120th amino acids) of human Hck, respectively, were obtained by PCR method.
SH2 Domain
- Sense Primer:: SEQ ID NO: 14
- Antisense Primer: SEQ ID NO: 4
SH3 Domain
- Sense Primer:: SEQ ID NO: 3
- Antisense Primer: SEQ ID NO: 15

The PCR products were ligated to the pAS-2 vector by the similar method to that described above to obtain Hck(SH2)/pAS-2 and Hck(SH3)/pAS-2, respectively.

With each of these plasmids, yeast Y190 strain was transformed by the method similar to that described in Example 1(3), and the cells were then transformed with Hck-c-58/pACT2 or with pACT2. Transformants were recovered after growing them on a medium of tryptophan(-) and leucine(-), and the binding of each of the three types of human Hck fragments (SH2 + SH3, SH2 and SH3) to HSB-1 was checked based on the β-galactosidase activity. The results are shown in Table 1. The yeast transformed with Hck-c-58/pACT2 and Hck(SH2+SH3)/pAS-2 was β-galactosidase activity positive, while the yeast transformed with Hck-c-58/pACT2 and Hck(SH2 )/pAS-2 was β-galactosidase activity negative. The yeast transformed with Hck(SH3)/pAS-2 became β-galactosidase activity positive by being transformed with pACT2 and the binding between the human Hck fragment and HSB-1 could not be judged. From these results, it is thought that HSB-1 does not bind to SH2 of human Hck but binds to the SH3 domain.

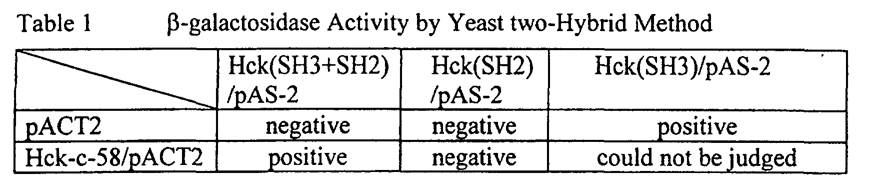

### Example 5 Deduction of Site in HSB-1, Which Binds to Human Hck

By a similar method to that for preparing the expression vector in Example 2, expression vectors which express HSB-1 and regions thereof (1st to 131st amino acids, and 70th to 404th amino acids in SEQ ID NO: 12), respectively, were prepared. cDNAs encoding 1st to 131st amino acids, and 70th to 404th amino acids in SEQ ID NO: 12, respectively, were prepared by PCR method, and the PCR products were inserted into an expression vector pcDNA4/HisMaxC (Invitrogen), respectively (the obtained plasmids are hereinafter referred to as "HSB-1/HisMaxC", "HSB-1(1-131)/HisMaxC" and "HSB-1(70-404)/HisMaxC", respectively).
PCR Primers for Preparing cDNA Encoding HSB-1(1-131)
- Sense Primer:: SEQ ID NO: 7
- Antisense Primer:: SEQ ID NO: 16
PCR Primers for Preparing cDNA Encoding HSB-1(70-404)
- Sense Primer:: SEQ ID NO: 17
- Antisense Primer:: SEQ ID NO: 8

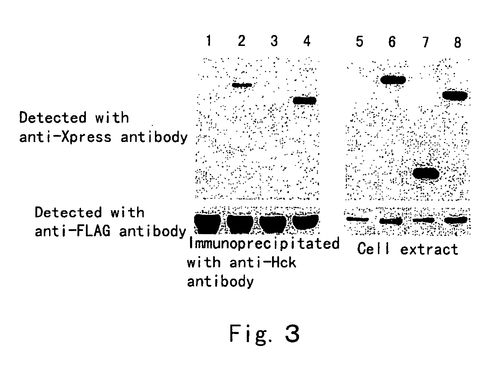

Then 293T cells were co-transformed with the two plasmids, Hck/pFLAG-CMV2 and, HSB-1/HisMaxC, HSB-1(1-131)/HisMaxC or HSB-1(70-404)/HisMaxC by the calcium phosphate method (using a kit commercially available from Prime). As a control, the cells were co-transformed with pcDNA4/HisMaxC and Hck/pFLAG-CMV2. After culturing the cells for 2 days, cells were collected and cell lysate solutions were prepared. The solutions were subjected to immunoprecipitation with anti-human Hck antibody, and the precipitated product was separated by SDS-polyacrylamide gel electrophoresis, followed by Western blotting and detection with anti-Xpress antibody. The results of the detection are shown in Fig. 3. Since HSB-1 and HSB-1(70-404) were detected by the anti-Xpress antibody and HSB-1(1-131) was not detected, it was confirmed that HSB-1 or HSB-1(70-404) (both of them were labelled with Xpress) bound to human Hck and immunoprecipitated with human Hck while HSB-1(1-131) did not bind to human Hck, so that it was not immunoprecipitated with human Hck.

### Example 6 Preparation of Transformant of CEM Cells, Which Consistently Produces HSB-1

The HSB-1/pEF/V5-HisC prepared in Example 3 was introduced into CEM cells by the electroporation method. The cells were cultured on a medium supplemented with an antibiotics G418 sulfate (purchased from Lifetech Oriental) (1.2 mg/ml), and clones (hereinafter referred to as CEM/HSB-1) expressing HSB-1 (labelled with V5) were selected from the grown resistant clones. Similarly, pEF/V5-HisC was introduced into CEM cells by the electroporation method. The cells were cultured on a medium supplemented with an antibiotics G418 sulfate (1.2 mg/ml), and grown resistant clones (hereinafter referred to as "CEM/pEF) were selected.

### Example 7 Measurement of Apoptosis Induced by TNF-α

The respective cells prepared in Example 6 were placed in wells of a 48-well microplate in an amount of 5000 cells/well, and human recombinant TNF-α was added to each of the wells to a final concentration of 0, 0.3, 1, 3 or 10 ng/ml. The cells were cultured at 37°C for 3 days and then the number of cells were counted. The counted numbers of the cells were expressed in terms of relative values taking the value obtained for the group to which TNF-α was not added as 100%. The results are shown in Fig. 4. Since apoptosis of CEM/HSB-1 cells was observed at lower concentrations than those observed for CEM cells and CEM/pEF cells, it is thought that HSB-1 promotes apoptosis of the CEM cells, which is induced by TNF-α.

## Claims

1. A protein which is the following (a) or (b):
(a) a protein having the amino acid sequence shown in SEQ ID NO:1;
(b) a protein having the same amino acid sequence as shown in SEQ ID NO: 1, except that one or several amino acids are deleted, substituted, inserted or added, which protein has an activity to bind to human tyrosine kinase Hck.

2. The protein according to claim 1, having the amino acid sequence shown in SEQ ID NO: 1 or SEQ ID NO:12.

3. A protein having the amino acid sequence which has a homology of not less than 80% to the amino acid sequence shown in SEQ ID NO: 1 or a region therein, which has an activity to bind to human tyrosine kinase Hck.

4. A nucleic acid encoding the protein according to any one of claims 1 to 3.

5. The nucleic acid according to claim 4, which has a nucleotide sequence shown in SEQ ID NO:2 or SEQ ID NO:13, or which has the same nucleotide sequence as shown in SEQ ID NO:2 or SEQ ID NO: 13, except that one or several nucleotides are deleted, substituted, inserted or added.

6. The nucleic acid according to claim 5, which has a nucleotide sequence shown in SEQ ID NO:2 or SEQ ID NO:13.

7. The nucleic acid according to claim 4, which has a homology of not less than 70% to the nucleotide sequence shown in SEQ ID NO:2 or SEQ ID NO:3.

8. The nucleic acid according to claim 4, which hybridizes with the nucleic acid having the nucleotide sequence shown in SEQ ID NO:2 or SEQ ID NO:13 under stringent conditions.

9. A recombinant vector containing the nucleic acid according to any one of claims 4 to 8, which can express the protein according to any one of claims 1 to 3 in a host cell.

10. A transformant transformed with the recombinant vector according to claim 9, which produces the protein according to any one of claims 1 to 3.


SEQUENCE LISTING
<110> SSP CO., LTD.
<120> Human Protein HSB-1 Which Binds to Human Tyrosine Kinase Hck and
Gene Encoding the Same
<130> 00PF213-PCT
<150> JP 11-309957
<151> 1999-10-29
<160> 17

<210> 1
<211> 404
<212> PRT
<213> Homo Sapience
<400> 1
Met Asp Ser Arg Thr Lys Ser Lys Asp Tyr Cys Lys Val Ile Phe Pro
1               5                   10                  15
Tyr Glu Ala Gln Asn Asp Asp Glu Leu Thr Ile Lys Glu Gly Asp Ile
            20                  25                  30
Val Thr Leu Ile Asn Lys Asp Cys Ile Asp Val Gly Trp Trp Glu Gly
        35                  40                  45
Glu Leu Asn Gly Arg Arg Gly Val Phe Pro Asp Asn Phe Val Lys Leu
    50                  55                  60
Leu Pro Pro Asp Phe Glu Lys Glu Gly Asn Arg Pro Lys Lys Pro Pro
65                  70                  75                  80
Pro Pro Ser Ala Pro Val Ile Lys Gln Gly Ala Gly Thr Thr Glu Arg
                85                  90                  95
Lys His Glu Ile Lys Lys Ile Pro Pro Glu Arg Pro Glu Met Leu Pro

            100                 105                 110
Asn Arg Thr Glu Glu Lys Glu Arg Pro Glu Arg Glu Pro Lys Leu Asp
        115                 120                 125
Leu Gln Lys Pro Ser Val Pro Ala Ile Pro Pro Lys Lys Pro Arg Pro
    130                 135                 140
Pro Lys Thr Asn Ser Leu Ser Arg Pro Gly Ala Leu Pro Pro Arg Arg
145                 150                 155                 160
Pro Glu Arg Pro Val Gly Pro Leu Thr His Thr Arg Gly Asp Ser Pro
                165                 170                 175
Lys Ile Asp Leu Ala Gly Ser Ser Leu Ser Gly Ile Leu Asp Lys Asp
            180                 185                 190
Leu Ser Asp Arg Ser Asn Asp Ile Asp Leu Glu Gly Phe Asp Ser Val
        195                 200                 205
Val Ser Ser Thr Glu Lys Leu Ser His Pro Thr Thr Ser Arg Pro Lys
    210                 215                 220
Ala Thr Gly Arg Arg Pro Pro Ser Gln Ser Leu Thr Ser Ser Ser Leu
225                 230                 235                 240
Ser Ser Pro Asp Ile Phe Asp Ser Pro Ser Pro Glu Glu Asp Lys Glu
                245                 250                 255
Glu His Ile Ser Leu Ala His Arg Gly Val Asp Ala Ser Lys Lys Thr
            260                 265                 270
Ser Lys Thr Val Thr Ile Ser Gln Val Ser Asp Asn Lys Ala Ser Leu
        275                 280                 285
Pro Pro Lys Pro Gly Thr Met Ala Ala Gly Gly Gly Gly Pro Ala Pro
    290                 295                 300
Leu Ser Ser Ala Ala Pro Ser Pro Leu Ser Ser Ser Leu Gly Thr Ala
305                 310                 315                 320

Gly His Arg Ala Asn Ser Pro Ser Leu Phe Gly Thr Glu Gly Lys Pro
                325                 330                 335
Lys Met Glu Pro Ala Ala Ser Ser Gln Ala Ala Val Glu Glu Leu Arg
            340                 345                 350
Thr Gln Val Arg Glu Leu Arg Ser Ile Ile Glu Thr Met Lys Asp Gln
        355                 360                 365
Gln Lys Arg Glu Ile Lys Gln Leu Leu Ser Glu Leu Asp Glu Glu Lys
    370                 375                 380
Lys Ile Arg Leu Arg Leu Gln Met Glu Val Asn Asp Ile Lys Lys Ala
385                 390                 395                 400
Leu Gln Ser Lys

<210> 2
<211> 1215
<212> DNA
<213> Homo Sapience
<400> 2
atg gac agc agg aca aag agc aag gat tac tgc aaa gta ata ttt cca 48
Met Asp Ser Arg Thr Lys Ser Lys Asp Tyr Cys Lys Val Ile Phe Pro
1               5                   10                  15
tat gag gca cag aat gat gat gaa ttg aca atc aaa gaa gga gat ata 96
Tyr Glu Ala Gln Asn Asp Asp Glu Leu Thr Ile Lys Glu Gly Asp Ile
            20                  25                  30
gtc act ctc atc aat aag gac tgc atc gac gta ggc tgg tgg gaa gga 144
Val Thr Leu Ile Asn Lys Asp Cys Ile Asp Val Gly Trp Trp Glu Gly
        35                  40                  45
gag ctg aac ggc aga cga ggc gtg ttc ccc gat aac ttc gtg aag tta 192

Glu Leu Asn Gly Arg Arg Gly Val Phe Pro Asp Asn Phe Val Lys Leu
    50                  55                  60
ctt cca ccg gac ttt gaa aag gaa ggg aat aga ccc aag aag cca ccg 240
Leu Pro Pro Asp Phe Glu Lys Glu Gly Asn Arg Pro Lys Lys Pro Pro
65                  70                  75                  80
cct cca tcc gct cct gtc atc aaa caa ggg gca ggc acc act gag aga 288
Pro Pro Ser Ala Pro Val Ile Lys Gln Gly Ala Gly Thr Thr Glu Arg
                85                  90                  95
aaa cat gaa att aaa aag ata cct cct gaa aga cca gaa atg ctt cca 336
Lys His Glu Ile Lys Lys Ile Pro Pro Glu Arg Pro Glu Met Leu Pro
            100                 105                 110
aac aga aca gaa gaa aaa gaa aga cca gag aga gag cca aaa ctg gat 384
Asn Arg Thr Glu Glu Lys Glu Arg Pro Glu Arg Glu Pro Lys Leu Asp
        115                 120                 125
tta cag aag ccc tcc gtt cct gcc ata ccg cca aaa aag cct cgg cca 432
Leu Gln Lys Pro Ser Val Pro Ala Ile Pro Pro Lys Lys Pro Arg Pro
    130                 135                 140
cct aag acc aat tct ctc agc aga cct ggc gca ctg ccc ccg aga agg 480
Pro Lys Thr Asn Ser Leu Ser Arg Pro Gly Ala Leu Pro Pro Arg Arg
145                 150                 155                 160
ccg gag aga ccg gtg ggt ccg ctg aca cac acc agg ggt gac agt cca 528
Pro Glu Arg Pro Val Gly Pro Leu Thr His Thr Arg Gly Asp Ser Pro
                165                 170                 175
aag att gac ttg gcc ggc agt tcg cta tct ggc atc ctg gac aaa gat 576
Lys Ile Asp Leu Ala Gly Ser Ser Leu Ser Gly Ile Leu Asp Lys Asp
            180                 185                 190
ctc tcg gac cgc agc aat gac att gac tta gaa ggt ttt gac tcc gtg 624

Leu Ser Asp Arg Ser Asn Asp Ile Asp Leu Glu Gly Phe Asp Ser Val
        195                 200                 205
gta tca tct act gag aaa ctc agt cat ccg acc aca agc aga cca aaa 672
Val Ser Ser Thr Glu Lys Leu Ser His Pro Thr Thr Ser Arg Pro Lys
    210                 215                 220
gct aca ggg agg cgg cct ccg tcc cag tcc ctc aca tct tca tcc ctt 720
Ala Thr Gly Arg Arg Pro Pro Ser Gln Ser Leu Thr Ser Ser Ser Leu
225                 230                 235                 240
tca agc cct gat atc ttc gac tcc cca agt ccc gaa gag gat aag gag 768
Ser Ser Pro Asp Ile Phe Asp Ser Pro Ser Pro Glu Glu Asp Lys Glu
                245                 250                 255
gaa cac att tca ctt gcg cac aga gga gtg gac gcg tca aag aaa act 816
Glu His Ile Ser Leu Ala His Arg Gly Val Asp Ala Ser Lys Lys Thr
            260                 265                 270
tcc aag act gtt acc ata tcc caa gtg tct gac aac aaa gca tcc ctg 864
Ser Lys Thr Val Thr Ile Ser Gln Val Ser Asp Asn Lys Ala Ser Leu
        275                 280                 285
ccg ccc aag ccg ggg acc atg gca gca ggt ggc ggt ggg cca gcc cct 912
Pro Pro Lys Pro Gly Thr Met Ala Ala Gly Gly Gly Gly Pro Ala Pro
    290                 295                 300
ctg tcc tca gcg gcg ccc tcc ccc ctg tca tcc tct ttg gga aca gct 960
Leu Ser Ser Ala Ala Pro Ser Pro Leu Ser Ser Ser Leu Gly Thr Ala
305                 310                 315                 320
gga cac aga gcc aac tcc ccg tct ctg ttc ggc acg gaa gga aaa cca 1008
Gly His Arg Ala Asn Ser Pro Ser Leu Phe Gly Thr Glu Gly Lys Pro
                325                 330                 335
aag atg gag cct gcg gcc agc agc cag gcg gcc gtg gag gag cta agg 1056

Lys Met Glu Pro Ala Ala Ser Ser Gln Ala Ala Val Glu Glu Leu Arg
            340                 345                 350
aca cag gtc cgc gag ctg agg agc atc atc gag acc atg aag gac cag 1104
Thr Gln Val Arg Glu Leu Arg Ser Ile Ile Glu Thr Met Lys Asp Gln
        355                 360                 365
cag aaa cga gag att aaa cag tta ttg tct gag ttg gat gaa gag aag 1152
Gln Lys Arg Glu Ile Lys Gln Leu Leu Ser Glu Leu Asp Glu Glu Lys
    370                 375                 380
aaa atc cgg ctt cgg ttg cag atg gaa gtg aac gac ata aag aaa gct 1200
Lys Ile Arg Leu Arg Leu Gln Met Glu Val Asn Asp Ile Lys Lys Ala
385                 390                 395                 400
cta caa tca aaa tga 1215
Leu Gln Ser Lys

<210> 3
<211> 32
<212> DNA
<213> Artificial Sequence
<220>
<223> Nucleic acid used as sense primer of PCR for amplifying SH2 domai
n and SH3 domain of human tyrosine kinase Hck
<400> 3
aagaattcgt ggttgccctg tatgattacg ag 32

<210> 4
<211> 32
<212> DNA

<213>  Artificial Sequence

<220>

<223>  Nucleic acid used as antisense primer of PCR for amplifying SH2 d

omain and SH3 domain of human tyrosine kinase Hck

<400>  4

aagtcgaccg acagtttctg gcagagcccg tc                                    32


<210>  5

<211>  30

<212>  DNA

<213>  Artificial Sequence

<220>

<223>  Nucleic acid used as sense primer of PCR for amplifying N-

terminal region of HSB-1

<400>  5

gacaccagac caactggtaa tggtagcgac                                       30


<210>  6

<211>  30

<212>  DNA

<213>  Artificial Sequence

<220>

<223>  Nucleic acid used as antisense primer of PCR for amplifying N-

terminal region of HSB-1

<400>  6

GAAGGCACAT GGCTGAATAT CGACGGTTTC                                       30

<210> 7

<211> 35

<212> DNA

<213> Artificial Sequence

<220>

<223> Nucleic acid used as sense primer of PCR for amplifying DNA

encoding HSB-1

<400> 7

aaggatccac agaaatggac agcaggacaa agagc 35

<210> 8

<211> 39

<212> DNA

<213> Artificial Sequence

<220>

<223> Nucleic acid used as antisense primer of PCR for amplifying DNA

encoding HSB-1

<400> 8

aagaattcct tttgattgta gagctttctt tatgtcgtt 39

<210> 9

<211> 34

<212> DNA

<213> Artificial Sequence

<220>

<223> Nucleic acid used as sense primer of PCR for amplifying DNA

encoding human tyrosine kinase Hck

<400>  9
gaattccatg gggtgcatga agtccaagtt cctc                                    34

<210>  10
<211>  33
<212>  DNA
<213>  Artificial Sequence
<220>
<223>  Nucleic acid used as antisense primer of PCR for amplifying DNA
encoding human tyrosine kinase Hck
<400>  10
ctcgagtggc tgctgttggt actggctctc tgt                                     33

<210>  11
<211>  505
<212>  PRT
<213>  Homo Sapience
<400>  11
Met Gly Cys Met Lys Ser Lys Phe Leu Gln Val Gly Gly Asn Thr Phe
1               5                   10                  15
Ser Lys Thr Glu Thr Ser Ala Ser Pro His Cys Pro Val Tyr Val Pro
            20                  25                  30
Asp Pro Thr Ser Thr Ile Lys Pro Gly Pro Asn Ser His Asn Ser Asn
        35                  40                  45
Thr Pro Gly Ile Arg Glu Ala Gly Ser Glu Asp Ile Ile Val Val Ala
    50                  55                  60
Leu Tyr Asp Tyr Glu Ala Ile His His Glu Asp Leu Ser Phe Gln Lys

65                  70                  75                  80
Gly Asp Gln Met Val Val Leu Glu Glu Ser Gly Glu Trp Trp Lys Ala
                85                  90                  95
Arg Ser Leu Ala Thr Arg Lys Glu Gly Tyr Ile Pro Ser Asn Tyr Val
            100                 105                 110
Ala Arg Val Asp Ser Leu Glu Thr Glu Glu Trp Phe Phe Lys Gly Ile
        115                 120                 125
Ser Arg Lys Asp Ala Glu Arg Gln Leu Leu Ala Pro Gly Asn Met Leu
    130                 135                 140
Gly Ser Phe Met Ile Arg Asp Ser Glu Thr Thr Lys Gly Ser Tyr Ser
145                 150                 155                 160
Leu Ser Val Arg Asp Tyr Asp Pro Arg Gln Gly Asp Thr Val Lys His
                165                 170                 175
Tyr Lys Ile Arg Thr Leu Asp Asn Gly Gly Phe Tyr Ile Ser Pro Arg
            180                 185                 190
Ser Thr Phe Ser Thr Leu Gln Glu Leu Val Asp His Tyr Lys Lys Gly
        195                 200                 205
Asn Asp Gly Leu Cys Gln Lys Leu Ser Val Pro Cys Met Ser Ser Lys
    210                 215                 220
Pro Gln Lys Pro Trp Glu Lys Asp Ala Trp Glu Ile Pro Arg Glu Ser
225                 230                 235                 240
Leu Lys Leu Glu Lys Lys Leu Gly Ala Gly Gln Phe Gly Glu Val Trp
                245                 250                 255
Met Ala Thr Tyr Asn Lys His Thr Lys Val Ala Val Lys Thr Met Lys
            260                 265                 270
Pro Gly Ser Met Ser Val Glu Ala Phe Leu Ala Glu Ala Asn Val Met
        275                 280                 285

Lys Thr Leu Gln His Asp Lys Leu Val Lys Leu His Ala Val Val Thr
    290                 295                 300
Lys Glu Pro Ile Tyr Ile Ile Thr Glu Phe Met Ala Lys Gly Ser Leu
305                 310                 315                 320
Leu Asp Phe Leu Lys Ser Asp Glu Gly Ser Lys Gln Pro Leu Pro Lys
                325                 330                 335
Leu Ile Asp Phe Ser Ala Gln Ile Ala Glu Gly Met Ala Phe Ile Glu
            340                 345                 350
Gln Arg Asn Tyr Ile His Arg Asp Leu Arg Ala Ala Asn Ile Leu Val
        355                 360                 365
Ser Ala Ser Leu Val Cys Lys Ile Ala Asp Phe Gly Leu Ala Arg Val
    370                 375                 380
Ile Glu Asp Asn Glu Tyr Thr Ala Arg Glu Gly Ala Lys Phe Pro Ile
385                 390                 395                 400
Lys Trp Thr Ala Pro Glu Ala Ile Asn Phe Gly Ser Phe Thr Ile Lys
                405                 410                 415
Ser Asp Val Trp Ser Phe Gly Ile Leu Leu Met Glu Ile Val Thr Tyr
            420                 425                 430
Gly Arg Ile Pro Tyr Pro Gly Met Ser Asn Pro Glu Val Ile Arg Ala
        435                 440                 445
Leu Glu Arg Gly Tyr Arg Met Pro Arg Pro Glu Asn Cys Pro Glu Glu
    450                 455                 460
Leu Tyr Asn Ile Met Met Arg Cys Trp Lys Asn Arg Pro Glu Glu Arg
465                 470                 475                 480
Pro Thr Phe Glu Tyr Ile Gln Ser Val Leu Asp Asp Phe Tyr Thr Ala
                485                 490                 495
Thr Glu Ser Gln Tyr Gln Gln Gln Pro

            500                 505

<210> 12
<211> 404
<212> PRT
<213> Homo Sapience
<400> 12

<210> 13
<211> 1215
<212> DNA
<213> Homo Sapience
<400> 13
atg gac agc agg aca aag agc aag gat tac tgc aaa gta ata ttt cca      48
tat gag gca cag aat gat gat gaa ttg aca atc aaa gaa gga gat ata      96
gtc act ctc atc aat aag gac tgc atc gac gta ggc tgg tgg gaa gga      144
gag ctg aac ggc aga cga ggc gtg ttc ccc gat aac ttc gtg aag tta      192
ctt cca ccg gac ttt gaa aag gaa ggg aat aga ccc aag aag cca ccg      240
cct cca tcc gct cct gtc atc aaa caa ggg gca ggc acc act gag aga      288
aaa cat gaa att aaa aag ata cct cct gaa aga cca gaa atg ctt cca 336
aac aga aca gaa gaa aaa gaa aga cta gag aga gag cca aaa ctg gat 384
tta cag aag ccc tcc gtt cct gcc ata ccg cca aaa aag cct cgg cca 432
cct aag acc aat tct ctc agc aga cct ggc gca ctg ccc ccg aga agg 480
ccg gag aga ccg gtg ggt ccg ctg aca cac acc agg ggt gac agt cca 528
aag att gac ttg gcc ggc agt tcg cta tct ggc atc ctg gac aaa gat 576
ctc tcg gac cgc agc aat gac att gac tta gaa ggt ttt gac tcc gtg 624
gta tca tct act gag aaa ctc agt cat ccg acc aca agc aga cca aaa 672
gct aca ggg agg cgg cct ccg tcc cag tcc ctc aca tct tca tcc ctt 720
tca agc cct gat atc ttc gac tcc cca agt ccc gaa gag gat aag gag     768
gaa cac att tca ctt gcg cac aga gga gtg gac gcg tca aag aaa act     816
tcc aag act gtt acc ata tcc caa gtg tct gac aac aaa gca tcc ctg     864
ccg ccc aag ccg ggg acc atg gca gca ggt ggc ggt ggg cca gcc cct     912
ctg tcc tca gcg gcg ccc tcc ccc ctg tca tcc tct ttg gga aca gct     960
gga cac aga gcc aac tcc ccg tct ctg ttc ggc acg gaa gga aaa cca     1008
aag atg gag cct gcg gcc agc agc cag gcg gcc gtg gag gag cta agg     1056
aca cag gtc cgc gag ctg agg agc atc atc gag acc atg aag gac cag     1104
cag aaa cga gag att aaa cag tta ttg tct gag ttg gat gaa gag aag     1152
aaa atc cgg ctt cgg ttg cag atg gaa gtg aac gac ata aag aaa gct      1200
cta caa tca aaa tga                                                  1215

<210>  14

<211>  32

<212>  DNA

<213>  Artificial Sequence

<220>

<223>  Nucleic acid used as sense primer of PCR for amplifying DNA

encoding SH2 domain of human tyrosine kinase Hck

<400>  14

aagaattcga ggagtggttt ttcaagggca tc                                  32

<210>  15

<211>  32

<212>  DNA

<213>  Artificial Sequence

<220>

<223>  Nucleic acid used as antisense primer of PCR for amplifying DNA

encoding SH3 domain of human tyrosine kinase Hck

<400>  15

aagtcgactg tctccagaga gtcaacgcgg gc                                  32

<210>  16

<211>  38

<212>  DNA

<213>  Artificial Sequence

<220>

<223>  Nucleic acid used as antisense primer of PCR amplifying DNA

encoding HSB1 (1-131)

<400>  16

aagaattcct tctgtaaatc cagttttggc tctctctc                                38

<210>  17

<211>  44

<212>  DNA

<213>  Artificial Sequence

<220>

<223>  Nucleic acid used as sense primer of PCR amplifying DNA encoding

HSB-1 (70-404)

<400>  17

aaggatccac catggaaaag gaagggaata gacccaagaa gcca                         44